(19) Europäisches Patentamt
European Patent Office
Office européen des brevets

(11) Publication number: **0 430 385 A2**

# (12) EUROPEAN PATENT APPLICATION

(21) Application number: **90250296.2**

(22) Date of filing: **29.11.90**

(51) Int. Cl.[5]: **C07D 471/04**, C07D 213/77, C07D 409/12, C07D 405/12, C07D 401/12, C07D 417/12, A01N 43/90, A01N 43/40, A01N 43/82, //(C07D471/04, 249:00,221:00)

(30) Priority: **30.11.89 DE 3939892**
**02.03.90 DE 4007025**
**11.04.90 DE 4012040**
**11.05.90 DE 4015647**

(43) Date of publication of application:
**05.06.91 Bulletin 91/23**

(84) Designated Contracting States:
**AT BE CH DE DK ES FR GB GR IT LI LU NL SE**
**Bulletin**

(71) Applicant: **SCHERING AKTIENGESELLSCHAFT**
**Berlin und Bergkamen**
**Müllerstrasse 170/178 Postfach 65 03 11**
**W-1000 Berlin 65(DE)**

(72) Inventor: **Geissler, Jens, Dr.**
**Schwendyweg 13**
**W-1000 Berlin 20(DE)**
Inventor: **Franke, Helga, Dr.**
**Spiessergasse 6b**
**W-1000 Berlin 27(DE)**
Inventor: **Angermann, Alfred, Dr.**
**Nussbaumallee 12**
**W-1000 Berlin 19(DE)**
Inventor: **Hömberger, Günter, Dr.**
**Waldmannstrasse 21**
**W-1000 Berlin 46(DE)**
Inventor: **Johann, Gerhard, Dr.**
**Hermsdorfer Damm 147**
**W-1000 Berlin 26(DE)**
Inventor: **Bohner, Jürgen, Dr.**
**Germendorfer Strasse 57**
**W-1000 Berlin 26(DE)**
Inventor: **Mertens, Rüdiger, Dr.**
**An der Wildbahn 88**
**W-1000 Berlin 27(DE)**
Inventor: **Rees, Richard, Dr.**
**Speerweg 8**
**W-1000 Berlin 28(DE)**

## (54) Substituted 1,2,4-triazolo-[4,3-a]pyridines, and substituted N'-(2-pyridyl)-hydrazones, and their use as herbicides.

(57) The invention provides herbicidal compositions comprising relates to new substituted 1,2,4-triazolo-[4,3-a]-pyridines of general formula I

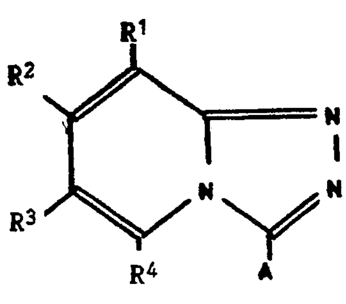

(I)

EP 0 430 385 A2

and substituted N'-(2-pyridyl)hydrazones of general formula II

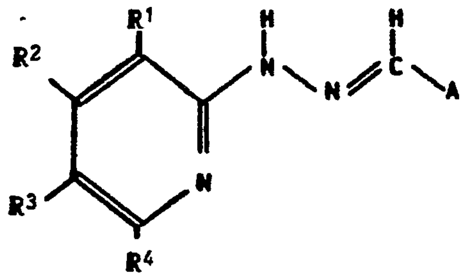

(II)

in which A and $R^{1-4}$ have the meanings given in the description. Many compounds are novel and form part of the invention.

# SUBSTITUTED 1,2,4-TRIAZOLO[4,3-A]PYRIDINES AND SUBSTITUTED N'-(2-PYRIDYL)ALDEHYDE HYDRAZONES AND THEIR USE AS HERBICIDES

This invention relates to substituted 1,2,4-triazolo-[4,3-a]pyridines, and substituted N'-(2-pyridyl)-hydrazones, and their use as herbicides.

It is known that certain 1,2,4-triazolo[4,3-a]pyridines and substituted N'-(2-pyridyl)hydrazones have pharmacological properties (EP 210 648 and US 4 121 036). However herbicidal activity for these compounds was not known.

We have now found that substituted 1,2,4-triazolo-[4,3-a]pyridines of general formula I

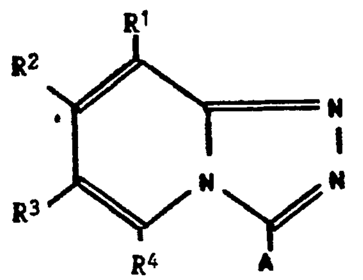

(I)

and substituted N'-(2-pyridyl)hydrazones of general formula II

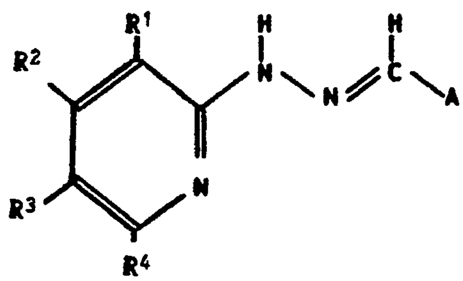

(II)

in which

A is a 5-membered heterocyclic group, comprising 1 to 3 hetero atoms selected from N, O and S, in which any ring carbon is optionally substituted by halogen, $C_{1-4}$-alkyl or halo-$C_{1-4}$-alkyl and one ring nitrogen is optionally substituted by $C_{1-4}$-alkyl, or

A is $C_{1-10}$-alkyl, $C_{2-10}$-alkenyl or $C_{2-10}$-alkynyl, each of which is optionally substituted by one or more of the same or different halogen, cyano, $C_{3-6}$-cycloalkyl, halo-$C_{3-6}$-cycloalkyl, cyano-$C_{3-6}$-cycloalkyl, $C_{1-4}$-alkyl-$C_{3-6}$-cycloalkyl, halo-$C_{1-4}$-alkyl-$C_{3-6}$-cycloalkyl, cyano-$C_{1-4}$-alkyl-$C_{3-6}$-cycloalkyl, $C_{3-6}$-cycloalkenyl, halo-$C_{3-6}$-cycloalkenyl, cyano-$C_{3-6}$-cycloalkenyl, $C_{1-4}$-alkyl-$C_{3-6}$-cycloalkenyl, halo-$C_{1-4}$-alkyl-$C_{3-6}$-cycloalkenyl or cyano-$C_{1-4}$-alkyl-$C_{3-6}$-cycloalkenyl, $C_{3-8}$-cycloalkyl or $C_{3-8}$-cycloalkenyl, each of which is optionally substituted by one or more of the same or different halogen; cyano, $C_{1-4}$-alkyl, halo-$C_{1-4}$-alkyl, cyano-$C_{1-4}$-alkyl, $C_{2-4}$-alkenyl, halo-$C_{2-4}$-alkenyl, cyano-$C_{2-4}$-alkenyl, $C_{2-4}$-alkynyl, halo-$C_{2-4}$-alkynyl or cyano-$C_{2-4}$-alkynyl, phenyl or benzyl, each of which is optionally substituted by one or more of the same or different halogen, hydroxy, $C_{1-6}$-alkyl, halo-$C_{1-6}$-alkyl, $C_{3-6}$-cycloalkyl, halo-$C_{3-6}$-cycloalkyl, phenyl-$C_{1-6}$-alkyl, $C_{2-6}$-alkenyl, halo-$C_{2-6}$-alkenyl, phenyl-$C_{2-6}$-alkenyl, $C_{2-6}$-alkynyl, halo-$C_{2-6}$-alkynyl, phenyl-$C_{2-6}$-alkynyl, $C_{1-6}$-alkoxy, halo-$C_{1-6}$-alkoxy, $C_{1-4}$-alkoxy-$C_{1-6}$-alkoxy, $C_{1-4}$-alkylamino-$C_{1-6}$-alkoxy, di-$C_{1-4}$-alkylamino-$C_{1-6}$-alkoxy, $C_{2-6}$-alkenyloxy, mercapto, $C_{1-6}$-alkylthio, halo-$C_{1-6}$-alkylthio, $C_{1-6}$-alkylsulphinyl, $C_{1-6}$-alkylsulphonyl, $C_{1-6}$-alkanoyl, halo-$C_{1-6}$-alkanoyl, $C_{1-6}$-alkoxycarbonyl, phenyl, halophenyl, $C_{1-6}$-alkylphenyl, phenoxy, halophenoxy, $C_{1-6}$-alkylphenoxy, nitrophenoxy, amino, $C_{1-4}$-alkylamino, di-$C_{1-4}$-alkylamino, anilino, haloanilino, $C_{1-6}$-alkylanilino, cyano, nitro, methylenedioxy, ethylenedioxy, isopropylidenedioxy, or naphthyl or naphthylmethyl, each of which is optionally substituted by one or more of the same or different halogen, hydroxy, $C_{1-4}$-alkyl,

$C_{1-4}$-alkoxy, amino, $C_{1-4}$-alkylamino, di-$C_{1-4}$-alkylamino, cyano or nitro,

$R^1$ is halogen, hydroxy, $C_{1-6}$-alkyl, halo-$C_{1-6}$-alkyl, $C_{1-6}$-alkoxy, halo-$C_{1-6}$-alkoxy, $C_{1-6}$-alkylthio, halo-$C_{1-6}$-alkylthio, $C_{1-6}$-alkylsulphinyl, $C_{1-6}$-alkylsulphonyl, $C_{1-6}$-alkoxycarbonyl, phenyl-$C_{1-6}$-alkoxycarbonyl, phenyl, phenoxy, $C_{1-4}$-alkylamino, di-$C_{1-4}$-alkylamino, nitro or cyano, and

$R^2$, $R^3$ and $R^4$, which may be the same or different, have the same meaning as $R^1$ or are hydrogen,

have herbicidal activity and the invention accordingly provides herbicidal compositions comprising these compounds in admixture with an agiculturally acceptable diluent or carrier.

Many of these compounds are novel and the invention thus includes compounds of formula I, in which A and $R^{1-4}$ are as defined above, with the proviso that

a) when A is phenyl, $R^1$ is not bromo or methyl,
b) when A is 4-methoxyphenyl, $R^1$ is not nitro,
c) when A is methyl, $R^1$ is not bromo, amino, nitro or methyl, or
d) when A is ethyl, $R^1$ is not methyl; and

compounds of formula II, in which A and $R^{1-4}$ are as defined above, with the proviso that

a) when A is phenyl, $R^1$ is not bromo,
b) when A is phenyl and $R^2$ is methyl, $R^3$ is not nitro, or
c) when A is phenyl and $R^1$ and $R^3$ are chloro, $R^4$ is not methyl.

A preferred group of compounds are those of formula I in which

$R^1$ is halogen, especially chlorine or bromine, or trifluoromethyl;
$R^2$ is hydrogen;
$R^3$ is hydrogen, trifluoromethyl or halogen, especially bromine or chlorine;
$R^4$ is hydrogen or halogen, especially chlorine;
A is thienyl, furyl, pyrrolyl, imidazolyl or 1,2,3-thiadiazolyl, each of which is optionally substituted by $C_{1-4}$-alkyl, preferably methyl; $C_{4-6}$-alkyl, $C_{3-5}$-alkenyl or $C_{3-8}$-cycloalkyl, each of which is optionally substituted by halogen, especially chlorine; phenyl or benzyl, each of which is optionally substituted by one or more of the same or different halogen, nitro, methyl, methylthio, methoxy, trifluoromethyl, methoxycarbonyl, dimethylamino, phenyl or phenoxy; or is naphthyl.

In a particularly preferred group of compounds

$R^1$ is bromine or trifluoromethyl;
$R^2$ and $R^4$ are hydrogen; and
$R^3$ is hydrogen or bromine, especially hydrogen;
A is 2- or 3-thienyl, optionally substituted by methyl, 5-methylfuryl, N-methylpyrrolyl or 4-methyl-1,2,3-thiadiazolyl.

The term "halogen" means fluorine, chlorine, bromine and iodine.

The term "haloalkyl" or "haloalkyl" means that one or more hydrogen atoms are replaced by fluorine, chlorine, bromine or iodine.

It is to be understood that the term "alkyl", "alkenyl" and "alkynyl" includes branched as well as straight chained hydrocarbon groups.

The compounds of general formula II can exist also as tautomers of general formula II'

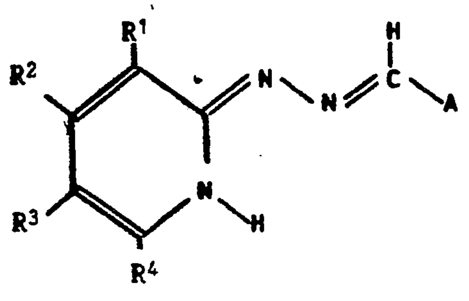

(II')

or as isomers of general formula II" and II'"

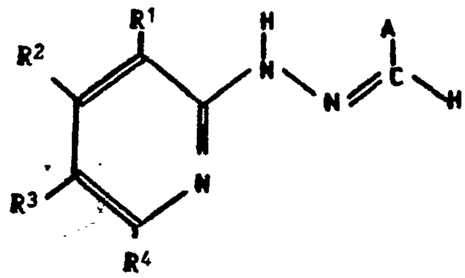

(II'')

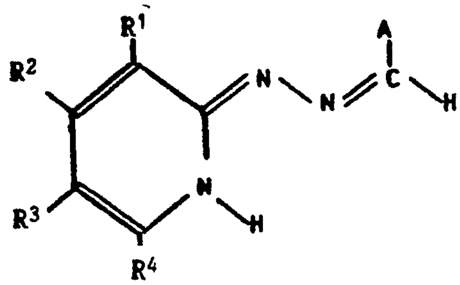

(II''')

These structures are also within the scope of the invention but for the sake of simplicity only the structures of general formula II are indicated.

The preparation of the compounds of general formula I can be carried by known methods, for example in a similar manner to that described in Tetrahedron 19 (1963) 1587, by reacting a compound of general formula II

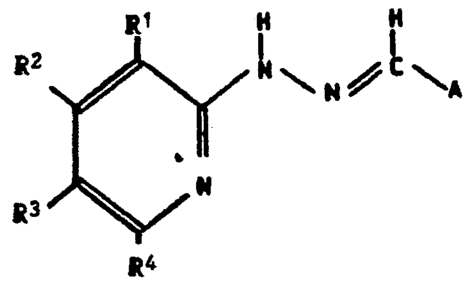

(II)

in which A, $R^1$, $R^2$, $R^3$ and $R^4$ have the meanings given under general formula I, with an oxidising agent. Alternatively the compounds can be prepared by other known methods, for example in a similar to those to those decribed in J. Org. Chem. 31 (1966) 251, Chem. Ber. 103 (1970) 1918 and J. Heterocycl. Chem. 15 - (1978) 439, by reacting a compound of general formula IIa

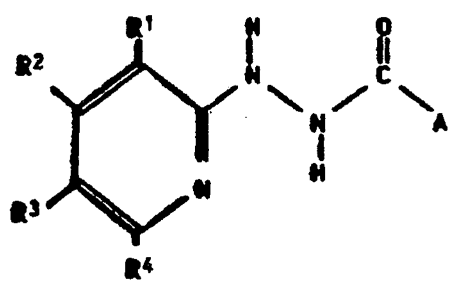

(IIa)

in which A, $R^1$, $R^2$, $R^3$ and $R^4$ have the meaning given under general formula I, with a halogenating agent.

Suitable halogenating agents include phosphorus oxychloride and thionyl chloride.

Suitable oxidising agents include lead tetraacetate and bromine.

The preparation of the compounds of general formula II can be carried out by known methods, for example in a similar manner to those described in J. Chem. Soc. 1915, 688, and Acta Chim. Scan B 37 -

(1983) 527, by reacting a compound of formula III

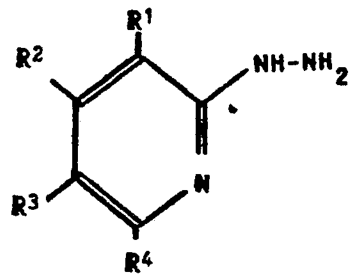

(III)

in which $R^1$, $R^2$, $R^3$ and $R^4$ have the meaning given under general formula I, with an aldehyde of general formula IV

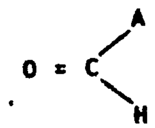

(IV)

in which A has the meaning given under general formula I in the presence of a suitable solvent.

The reactions can be carried out with or without a solvent. For this, there can be used any solvents or diluents which are inert to the reactants. Examples of such solvents or diluents are aliphatic, alicyclic and aromatic hydrocarbons, which can be optionally chlorinated, such as for example hexane, cyclohexane, petroleum ether, naphtha, benzene, toluene, methylene chloride, chloroform, carbon tetrachloride, ethylene dichloride, trichloroethane and chlorobenzene, ethers, such as for example diethyl ether, methyl ethyl ether, methyl t-butyl ether, diisopropyl ether, dibutyl ether, dioxane and tetrahydrofuran, ketones such as for example acetone, methyl ethyl ketone, methyl isopropyl ketone and methyl isobutyl ketone, nitriles, such as for example acetonitrile and propionitrile, alcohols, such as for example methanol, ethanol, isopropanol, butanol, tert-butanol, tert-amyl alcohol and ethylene glycol, esters, such as for example ethyl acetate and amyl acetate, amides, such as for example dimethylformamide and dimethylacetamide, sulphoxides, such as for example dimethyl sulphoxide and sulpholanes, such as for example sulpholane, and bases, such as for example pyridine and triethylamine.

All reactions are preferably carried out at atmospheric pressure, although higher or lower pressures can be used. The reactions can also be carried out over a wide temperature range. In general a temperature of between -20 °C and 200 °C is used.

The compounds of the invention prepared by these processes can be isolated from the reaction mixtures in conventional manner, for example by distillation of the solvent at normal or reduced pressure, by precipitation with water or by extraction. A higher level of purity can be achieved as a rule by column chromatography as well as by fractional distillation or crystallisation.

The compounds of the invention are, as a rule, almost colourless and odourless liquids or crystals, that are almost insoluble in water and and have limited solubility in aliphatic hydrocarbons such as petroleum ether, hexane, pentane and cyclohexane but are highly soluble in halogenated hydrocarbons, such as chloroform, methylene chloride and carbon tetrachloride, aromatic hydrocarbons such as benzene, toluene and xylene, ethers, such as diethyl ether, tetrahydrofuran and dioxane, nitriles, such as acetonitrile, alcohols, such as methanol and ethanol, amides, such as dimethylformamide, sulphoxides, such as dimethyl sulphoxide.

The preparation of compounds of general formula IIa can also be carried out in known manner. The hydrazines of general formula III as well as the aldehydes of general formula IV are either known in the literature or can be prepared by known literature methods. Suitable methods are described for example in J. Org. Chem. 27, (1962) 3965, for the hydrazines, or Synthesis 1988, 394 for the aldehydes.

The compounds of the invention show a good herbicidal activity against broad leaved weeds and grasses. A selective use of the compounds of the invention in various crops is possible for example in rape, beet, soya beans, cotton, rice, barley, wheat and other cereals. Individual active substances are particularly suitable as selective herbicides in beet, cotton, soya and cereals. However the compounds can be used for control of weeds in permanent crops, such as for example forestry, ornamental trees, fruit, vine, citrus, nut,

banana, coffee, tea, rubber, oil palm, cocoa, berry fruit and hop plantations and for the selective control of weeds in annual crops.

The compounds of the invention can used for example against the following plant species:

Dicotyledonous weeds of the species Sinapis, Lepidium, Galium, Stellaria, Matricaria, Anthemis, Galinsoga, Chenopodium, Brassica, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Lamium, Veronica, Abutilon, Datura, Viola, Galeopsis, Papaver, Centaurea and Chrysanthemum.

Monocotyledonous weeds of the species Avena, Alopecurus, Echinochloa, Setaria, Panicum, Digitaria, Poa, Eleusine, Brachiaria, Lolium, Bromus, Cyperus, Agropyron, Sagittaria, Monocharia, Fimbristylis, Eleocharis, Ischaemum and Apera.

The rates of use vary depending on the manner of pre- and postemergent use between 0.001 and 5 kg/ha.

The compounds of the invention can also be used as defoliants, desiccants and total herbicides.

The compounds of the invention can be used either alone or in admixture with one another or with other active agents. Optionally, other plant-protective agents or pesticides can be added, depending on the purpose for the treatment. When it is desired to broaden the spectrum of activity, other herbicides can also be added.

Herbicidally active mixing partners suitable in this connection include for example, the active agents listed in Weed Abstracts, vol. 39, No. 1, 1990, under the heading "Lists of common names and abbreviations employed for currently used herbicides and plant growth regulators in Weed Abstracts".

An improvement in the intensity and speed of action can be obtained, for example, by addition of suitable adjuvants, such as organic solvents, wetting agents and oils. Such additives may allow a decrease in the dose.

The designated active ingredients or their mixtures can suitably be used, for example, as powders, dusts, granules, solutions, emulsions or suspensions, with the addition of liquid and/or solid carriers and/or diluents and, optionally, binding, wetting, emulsifying and/or dispersing adjuvants.

Suitable liquid carriers are, for example aliphatic and aromatic hydrocarbons, such as benzene, toluene, xylene, cyclohexanone, isophorone, dimethyl sulphoxide, dimethylformamide and other mineral-oil fractions and plant oils.

Suitable solid carriers include mineral earths, e.g. bentonite, silica gel, talcum, kaolin, attapulgite, limestone, silicic acid and plant products, e.g. flours.

As surface-active agents there can be used for example calcium lignosulphonate, polyoxyethylenealkylphenyl ethers, naphthalene-sulphonic acids and their salts, phenolsulphonic acids and their salts, formaldehyde condensates, fatty alcohol sulphates, as well as substituted benzenesulphonic acids and their salts.

The percentage of the active ingredient(s) in the various preparations can vary within wide limits. For example, the compositions can contain about 10 to 90 percent by weight active ingredients, and about 90 to 10 percent by weight liquid or solid carriers, as well as, optionally up to 20 percent by weight of surfactant.

The agents can be applied in customary fashion, for example with water as the carrier in spray mixture volumes of approximately 100 to 1,000 l/ha. The agents can be applied using low-volume or ultra-low-volume techniques or in the form of so-called microgranules.

The preparation of these formulations can be carried out in known manner, for example by milling or mixing processes. Optionally, individual components can be mixed just before use for example by the so-called commonly used tank-mixing method.

Formulations can be prepared, for example, from the following ingredients.

A) Wettable Powder

1) 20 percent by weight active ingredient
68 percent by weight kaolin
10 percent by weight calcium lignosulphonate
2 percent by weight dialkylnaphthalene-sulphonate

2) 40 percent by weight active ingredient
25 percent by weight kaolin
25 percent by weight colloidal silicic acid
8 percent by weight calcium lignosulphonate
2 percent by weight sodium salt of N-methyl-N-oleyltaurine

B) Emulsifiable Concentrate

20 percent by weight active ingredient
75 percent by weight isophorone
2 percent by weight ethoxylated castor oil
3 percent by weight calcium dodecyl-benzenesulphonate

The following examples illustrate the preparation of compounds according to the invention of formula I.

Example 1
8-Chloro-3-(3-methyl-2-thienyl)-1,2,4-triazole-[4,3-a]pyridine.

A solution of 3.7g (23 mmol) bromine in 40 ml acetic acid was added dropwise, to a solution of 5.73g (22.9 mmol) N'-(3-chloro-2-pyridyl)-3-methyl-2-thiophenecarbaldehyde hydrazone and 5.95g (69 mmol) sodium acetate in 240 ml acetic acid. The mixture was stirred for one hour at room temperature and then added to 500 ml ice-water. The mixture was extracted with ethyl acetate, dried (magnesium sulphate), the solvent distilled and the residue recrystallised to give the title compound.

Yield: 1.9g = 33% of theory
Mp: 133-136 °C

In a similar manner the following compounds of formula I were obtained.

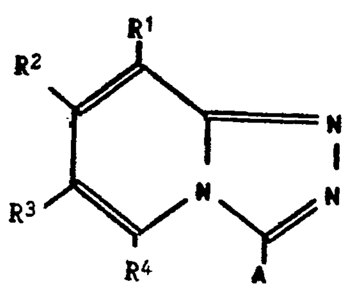

$R^2$ and $R^4$ = H

| Example | $R^1$ | $R^3$ | A | mp (°C) |
|---|---|---|---|---|
| 2 | Cl | H | 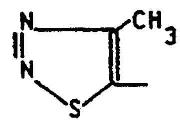 | 189-192 |
| 3 | $-CF_3$ | H | 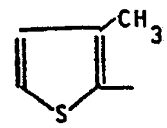 | 108-112 |

| Example | $R^1$ | $R^3$ | A | mp (°C) |
|---|---|---|---|---|
| 4 | Cl | H | 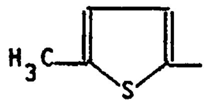 | 164-168 |
| 5 | Br | H | 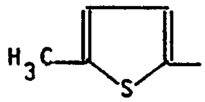 | 182-185 |
| 6 | Br | H | 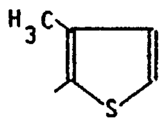 | 131-135 |
| 7 | $-CF_3$ | H | 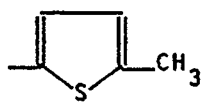 | 164-167 |
| 8 | $-CF_3$ | Br | 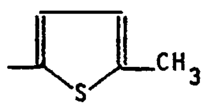 | 246 |
| 9 | $-CF_3$ | H | 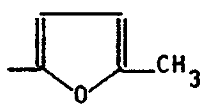 | 165-166 |
| 10 | Br | H | 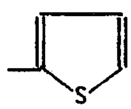 | 187-189 |
| 11 | Br | H | 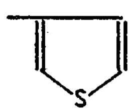 | 195-197 |
| 12 | Br | H | 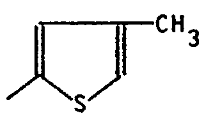 | 197-199 |

Example 13
8-Chloro-3-cyclopropyl-1,2,4-triazole[4,3-a]pyridine

3.5g (16.5 mmol N'-(3-Chloro-2-pyridyl)cyclopropane-carboxylic acid hydrazide was heated with 7.6 ml (81 mmol) phosphoros oxychloride for 6 hours under reflux. After cooling the reaction mixture, it was hydrolysed with saturated aqueous sodium hydrogen carbonate and extracted with ethyl acetate. The extract was dried (magnesium sulphate), the solvent distilled <u>in vacuo</u> and the residue recrystallised from ethyl acetate/hexane to give the title compound.

Yield: 1.2g = 37% of theory

Mp: 121-125 °C

In a similar manner to Examples 1 or 13 the following compounds were obtained.

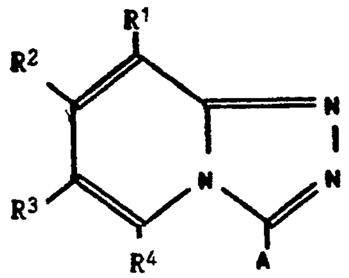

$R^2$, $R^3$ and $R^4$ = H

| Example | $R^1$ | A | mp (°C) |
|---|---|---|---|
| 14 | Cl | 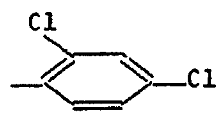 | 169 |
| 15 | Cl | 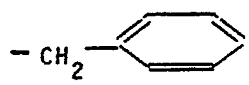 | 105-107 |
| 16 | Cl | t-Butyl | 172-174 |

| Example | $R^1$ | A | mp (°C) |
|---|---|---|---|
| 17 | Cl | 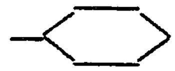 | 190-193 |
| 18 | $NO_2$ | $-C(CH_3)_2CH_2Cl$ | 191-193 |
| 19 | $-CF_3$ | 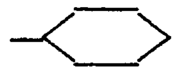 | 160-161 |
| 20 | $-CH_3$ | 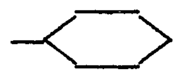 | 136-140 |
| 21 | Br | 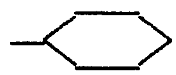 | 207-210 |
| 22 | Cl | $-CH(C_2H_5)_2$ | 71-75 |
| 23 | $-CF_3$ | $-C_6H_{13}$ | 78-80 |
| 24 | Cl | $-C_6H_{13}$ | 87-87,5 |
| 25 | $-CF_3$ | $-CH(C_2H_5)_2$ | 81-84 |
| 26 | $-CF_3$ | $-CH=C(CH_3)_2$ | 146-148 |

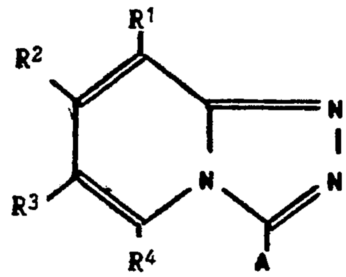

$R^2 = H$

| Example | $R^1$ | $R^3$ | $R^4$ | A | mp (°C) |
|---|---|---|---|---|---|
| 27 | $NO_2$ | H | H | 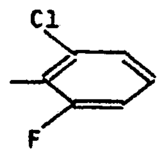 | 202-204 |
| 28 | Cl | H | H | 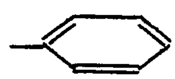 | 156-158 |
| 29 | Cl | H | H | 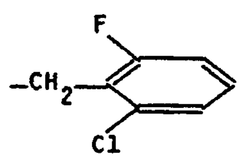 | 180-182 |
| 30 | $-NH_2$ | H | H | 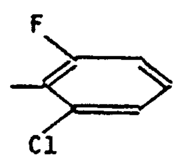 | 163-165 |
| 31 | Cl | H | H | 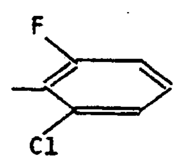 | 171-172 |
| 32 | Cl | $-CF_3$ | H | 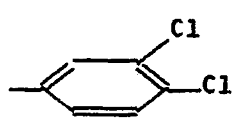 | 136 |

| Example | $R^1$ | $R^3$ | $R^4$ | A | mp (°C) |
|---|---|---|---|---|---|
| 33 | $-CF_3$ | Cl | H | 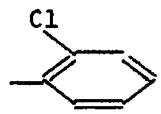 | 136 |
| 34 | Cl | H | H | 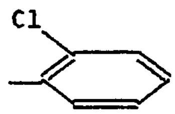 | 163 |
| 35 | Cl | H | H | 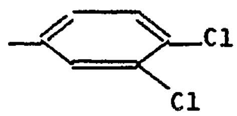 | 259 |
| 36 | Cl | $-CF_3$ | H | 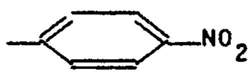 | 169 |
| 37 | Cl | $-CF_3$ | H | 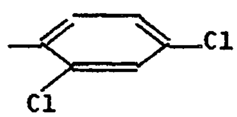 | 152 |
| 38 | Cl | $-CF_3$ | H | 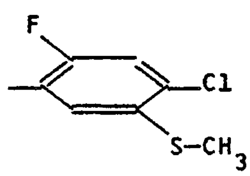 | 225 |
| 39 | Cl | H | H | 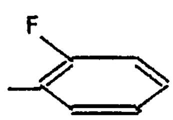 | 156-158 |
| 40 | Cl | H | H | 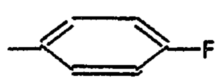 | 220 |

| Example | $R^1$ | $R^3$ | $R^4$ | A | mp (°C) |
|---|---|---|---|---|---|
| 41 | Cl | H | H | 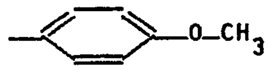 | 178-181 |
| 42 | Cl | H | Cl | 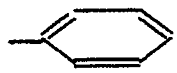 | 202-204 |
| 43 | $-CF_3$ | H | H | 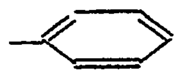 | 191-193 |
| 44 | Cl | Cl | H | 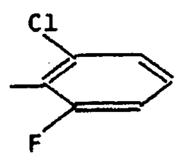 | 157-161 |
| 45 | Cl | H | H | 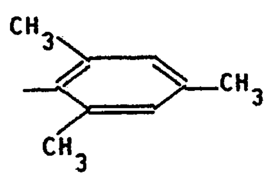 | 167-169 |
| 46 | $-NO_2$ | H | H | 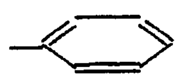 | 215-217 |
| 47 | Cl | H | H | 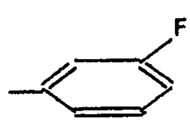 | 194-196 |
| 48 | Cl | H | H | 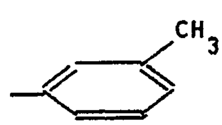 | 130-132 |

| Example | $R^1$ | $R^3$ | $R^4$ | A | mp (°C) |
|---|---|---|---|---|---|
| 49 | Cl | H | H | 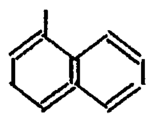 | 216-218 |
| 50 | Cl | Cl | H | 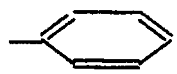 | 150 |
| 51 | Cl | $-CF_3$ | H | 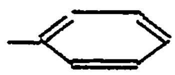 | 140-143 |
| 52 | Br | H | H | 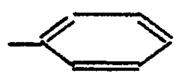 | 178-180 |
| 53 | $CH_3$ | H | H | 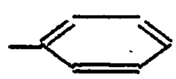 | 117-119 |
| 54 | Cl | H | H | 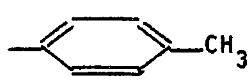 | 165-168 |
| 55 | Cl | H | H | 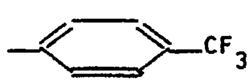 | 188-191 |

| Example | $R^1$ | $R^3$ | $R^4$ | A | mp (°C) |
| --- | --- | --- | --- | --- | --- |
| 56 | Cl | H | H | 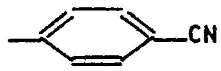 | >200 |
| 57 | $-CF_3$ | H | H | 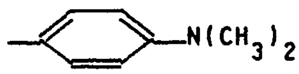 | 222-224 |
| 58 | $-CF_3$ | H | H | 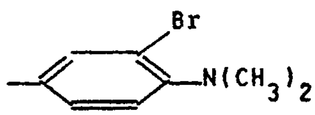 | 179 |
| 59 | $-CF_3$ | H | H | 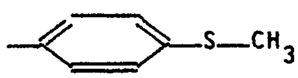 | 195-197 |
| 60 | $-CF_3$ | H | H | 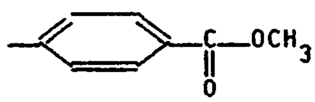 | 210-212 |
| 61 | Cl | H | H | 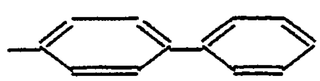 | 248-251 |
| 62 | I | H | H | 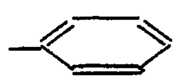 | 208 |
| 63 | $-CF_3$ | H | H | 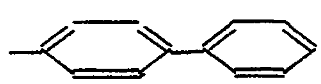 | 251 |

| Example | $R^1$ | $R^3$ | $R^4$ | A | mp (°C) |
|---|---|---|---|---|---|
| 64 | Br | H | H | 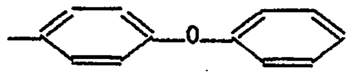 | 172-173 |
| 65 | Br | H | H | 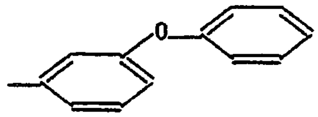 | 135-136 |
| 66 | $-CF_3$ | H | H | 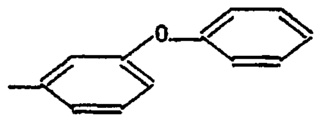 | 98-100 |
| 67 | Br | H | H | 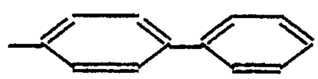 | 180 |
| 68 | $-CF_3$ | H | H | 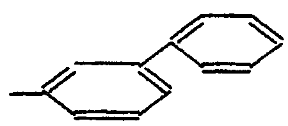 | 147-148 |

The following examples illustrate the preparation of compounds of general formula II.

Example 69
N'-(3-Chloro-2-pyridyl)-3-methyl-2-thiophenecarbaldehyde hydrazone

8g (55.7 mmol) 3-Chloro-2-hydrazinopyridine was stirred for 3 hours at room temperature with 7.03g (55.7 mmol) 3-methyl-2-thiophene-carboxaldehyde in 130 ml ethanol. The solvent was distilled in vacuo and the residue recrystallised from methanol to give the desired compound.

Yield: 8.73g = 62% of theory
Mp: > 200 °C (dec.)

In a similar manner the following compounds of formula II were obtained

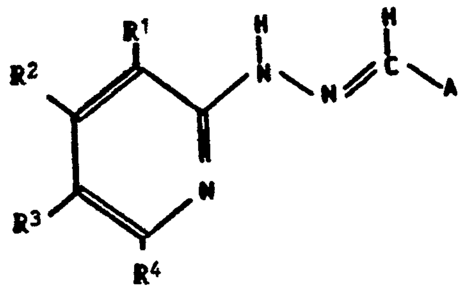

$R^2$, $R^3$ and $R^4$ = H

| Example | $R^1$ | A | mp (°C) |
|---|---|---|---|
| 70 | Cl | 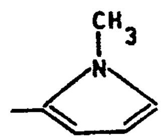 | 200-202 |
| 71 | $-CF_3$ | 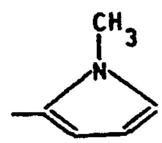 | 173-175 |
| 72 | $-CF_3$ | 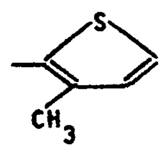 | 215 |

| Example | $R^1$ | A | mp (°C) |
|---|---|---|---|
| 73 | Br | 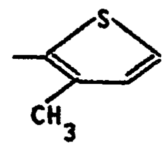 | ) 230 (decomposition) |
| 74 | $-CF_3$ | 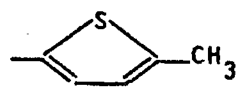 | 149-151 |
| 75 | Br | 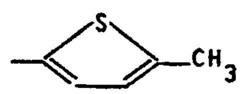 | 156-158 |
| 76 | Cl | 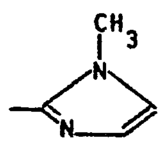 | 228-230 |
| 77 | Br | 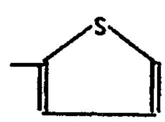 | 197-199 |
| 78 | Br | 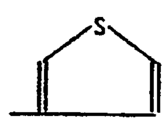 | 179-181 |

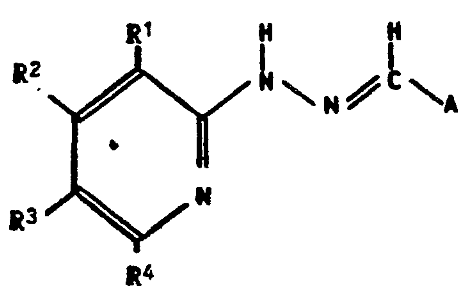

| Example | $R^1$ | $R^2$ | $R^3$ | $R^4$ | A | mp (°C) |
|---|---|---|---|---|---|---|
| 79 | $CF_3$ | H | H | H | 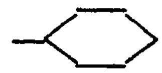 | 124-125 |
| 80 | Cl | H | H | H | 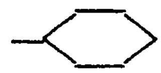 | 155-158 |
| 81 | Cl | H | H | H | 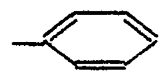 | 146-149 |
| 82 | Cl | H | H | H | 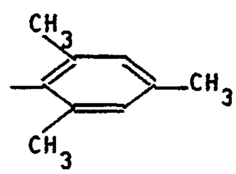 | 172-175 |

| Example | $R^1$ | $R^2$ | $R^3$ | $R^4$ | A | mp (°C) |
|---|---|---|---|---|---|---|
| 83 | $-CH_3$ | H | H | H | 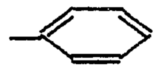 | 136-140 |
| 84 | Br | H | H | H | 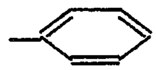 | 134-137 |
| 85 | Br | H | H | H | 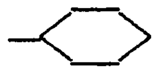 | 149-152 |
| 86 | $-CH_3$ | H | H | H | 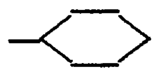 | 154-157 |
| 87 | Cl | H | H | H | t-Butyl | 143-145 |
| 88 | Cl | H | H | H | 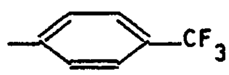 | 125-130 |
| 89 | Cl | H | H | H | 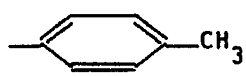 | 159-162 |
| 90 | Cl | H | H | H | $-CH(C_2H_5)_2$ | 140-142 |
| 91 | $-CF_3$ | H | H | H | $-CH(C_2H_5)_2$ | 81-83 |
| 92 | $-CF_3$ | H | H | H | 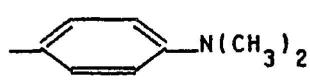 | 140-142 |

| Example | $R^1$ | $R^2$ | $R^3$ | $R^4$ | A | mp (°C) |
|---|---|---|---|---|---|---|
| 93 | H | H | $-CF_3$ | H | 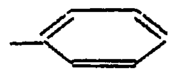 | 215 |
| 94 | $-CF_3$ | H | H | H | 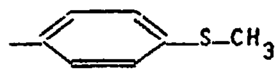 | 101-104 |
| 95 | Cl | H | H | H | $-C_6H_{13}$ | 60-62 |
| 96 | $-CF_3$ | H | H | H | $-C_6H_{13}$ | 76-78 |
| 97 | $-CF_3$ | H | H | H | 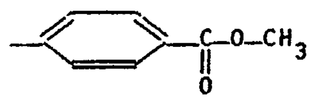 | 169-170 |
| 98 | Cl | H | H | H | 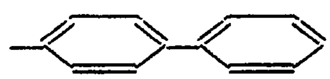 | 110-113 |
| 99 | -CN | $-CH_3$ | H | $CH_3$ | 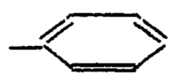 | dec. > 200 |
| 100 | $-CF_3$ | H | H | H | 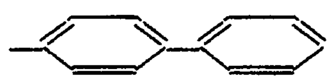 | 149-151 |
| 101 | Br | H | H | H | 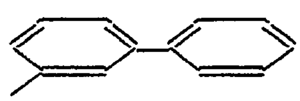 | 145-148 |
| 102 | $-CF_3$ | H | H | H | 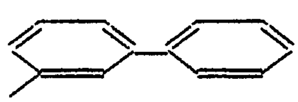 | 128-130 |
| 103 | Br | H | H | H | $-CH=C(CH_3)_2$ | 177-180 |
| 104 | $-CF_3$ | H | H | H | $-CH=C(CH_3)_2$ | 154-156 |

The following examples illustrate the possibilities for use of the compounds of the invention.

In these Examples the abbreviations have the following meanings:

| | |
|---|---|
| ABUTH | = Abutilon theophrasti |
| ALOMY | = Alopecurus myosuroides |
| CYPES | = Cyperus esculentus |
| ECHGH | = Echinochloa crus-galli |
| GALAP | = Galium aparine |
| GOSHI | = Gossypium hirsutum |
| IPOSS | = Ipomea purpurea |
| MATCH | = Matricaria chamomilla |
| MOOVA | = Monochoria vaginalis |
| ORYSA | = Oryza sativa |
| PANSS | = Panicum maximum |
| PASDS | = Paspalum distichum |
| POLSS | = Polygonum sp. |
| SEBEX | = Sesbania exaltata |
| SETVI | = Setaria viridis |
| SOLSS | = Solanum sp. |
| TRZAX | = Triticum aestivum |
| VERPE | = Veronica persica |
| ZEAMX | = Zea mays |

In these Examples damage to plants is assessed on a score of 0 to 4, in which the scores have the following meanings:

| | |
|---|---|
| 0 | = no damage |
| 1 | = 1 - 24% damage |
| 2 | = 25 - 74% damage |
| 3 | = 75 - 89% damage |
| 4 | = 90 - 100% damage |

**Example A**

In a greenhouse, the noted plant species were treated pre-emergently with the noted compounds, at a rate of 1.0 kg active ingredient/ha. The compounds were sprayed evenly over the soil as emulsions or suspensions in 500 litres water/ha. Three weeks after the treatment, the compounds of the invention showed a high crop selectivity in cotton with excellent activity against the weeds. The comparison material did not show the same high activity.

| Compound No. | GOSHI | GALAP | IPOSS | ALOMY |
|---|---|---|---|---|
| 1 | 0 | 4 | 4 | - |
| 2 | 0 | - | 4 | - |
| 3 | 0 | 4 | 4 | - |
| 6 | 0 | 3 | 4 | - |
| 10 | 0 | - | 3 | - |
| 11 | 0 | 4 | 3 | - |
| 69 | 0 | 3 | 4 | - |
| 71 | 0 | 3 | 4 | - |
| 72 | 0 | 3 | 4 | - |
| 73 | 0 | 4 | 4 | - |
| 79 | 0 | - | 4 | - |
| 81 | 1 | - | 4 | - |
| 83 | 0 | - | 4 | 3 |
| 84 | 0 | - | 4 | 4 |
| 85 | 0 | - | 4 | 4 |
| 86 | 0 | - | 4 | 3 |
| 87 | 1 | - | 4 | - |
| 90 | 0 | - | 4 | - |
| 91 | 0 | - | 3 | - |
| 103 | 0 | - | - | 4 |
| Untreated | 0 | 0 | 0 | 0 |
| Comparison | | | | |
| Methabenzthiazuron | 0 | 2 | 2 | - |
| Linuron | 0 | 2 | 1 | 1 |

**Example B**

In a greenhouse, the compounds noted in the table were applied at the rates mentioned. For this the formulated active ingredients were pipetted onto the water surface of vessels containing 1500 ml water. Test plants that were treated pre-emergently and in the 1 to 5 leaf stages. Three weeks after the application, the damage to the plants was assessed. The compounds of the invention showed good activity against important rice weeds, with good selectivity in paddy-rice.

| Compound No. | Water application kg AS/ha | ORYSA | ECHCG | MOOVA | PASDS | CYPSE |
|---|---|---|---|---|---|---|
| 6 | 0,25 | 0 | 4 | 4 | - | 4 |
| 84 | 1,0 | 1 | 4 | 3 | 4 | - |
| 85 | 1,0 | 1 | 3 | 3 | - | - |
| Untreated | 0 | 0 | 0 | 0 | 0 | 0 |
| Comparison | | | | | | |
| Mefenacet | 0,25 | 0 | 3 | 0 | - | 0 |

**Example C**

In a greenhouse, the noted plant species were treated pre-emergently with the noted compounds, at a rate of 0.1 kg active ingredient/ha. The compounds were sprayed evenly over the soil as emulsions or suspensions in 500 litres water/ha. Three weeks after the treatment, the compounds of the invention showed a high crop selectivity in wheat with excellent activity against the weeds. The comparison material did not show the same high activity.

| Compound No. | TRZAX | SETVI | PANSS | POLSS | VERPE |
|---|---|---|---|---|---|
| 16 | 1 | 2 | 2 | 2 | 4 |
| 17 | 1 | 3 | 4 | 3 | 4 |
| 21 | 1 | - | - | 3 | 3 |
| Untreated | 0 | 0 | 0 | 0 | 0 |
| Comparison | | | | | |
| Isoproturon | 2 | 2 | 2 | 2 | 2 |

**Example D**

In a greenhouse, the noted plant species were treated post-emergently with the noted compounds, at a rate of 1.0 kg active ingredient/ha. The compounds were sprayed evenly over the plants as emulsions or suspensions in 500 litres water/ha. Two weeks after the treatment, the compounds of the invention showed a high crop selectivity in cotton with excellent activity against the weeds. The comparison material did not show the same high selectivity.

| Compound No. | GOSHI | ABUTH | IPOSS | MATCH | POLSS | SOLSS | VERPE | SEBEX |
|---|---|---|---|---|---|---|---|---|
| 13 | 1 | 4 | 4 | 4 | - | - | - | - |
| 16 | 1 | 4 | 4 | 4 | 4 | 4 | 4 | 4 |
| 17 | 1 | 4 | 4 | 4 | 4 | 4 | 4 | 4 |
| 19 | 0 | - | 4 | 4 | - | 4 | 4 | 4 |
| 20 | 1 | 4 | 4 | 4 | - | 4 | 4 | - |
| 21 | 0 | 4 | 4 | 4 | 4 | 4 | 4 | 4 |
| 26 | 0 | 4 | 4 | 4 | - | - | - | - |
| 79 | 0 | 4 | 4 | 4 | - | - | - | - |
| 84 | 1 | 4 | 4 | 4 | - | - | - | - |
| 85 | 0 | 4 | 4 | 4 | - | - | - | - |
| 86 | 0 | 4 | 4 | 4 | - | - | - | - |
| 89 | 1 | 4 | 3 | 4 | - | - | - | - |
| 104 | 1 | 4 | - | - | - | - | - | - |
| Untreated | 0 | 0 | 0 | 0 | 0 | 0 | 0 | 0 |
| <u>Comparison</u> | | | | | | | | |
| | 3 | 4 | 4 | 4 | 4 | 4 | 3 | 4 |
| Linuron | 4 | 4 | 4 | 4 | 4 | 4 | 4 | 4 |
| Chlortoluron | 4 | 4 | 4 | 4 | - | - | - | - |

**Example E**

In a greenhouse, the noted plant species were treated post-emergently with the noted compounds, at a rate of 1.0 kg active ingredient/ha. The compounds were sprayed evenly over the plants as emulsions or suspensions in 500 litres water/ha. Three weeks after the treatment, the compounds of the invention showed a high crop selectivity in cotton and maize with excellent activity against the weeds. The comparison material did not show the same high selectivity.

| Compound No. | GOSHI | ZEAMX | ABUTH | IPOSS | MATCH | SEBEX | SOLSS |
|---|---|---|---|---|---|---|---|
| 28 | 1 | 0 | 4 | 4 | 4 | 4 | 4 |
| 29 | 1 | 1 | 4 | 4 | 4 | 4 | 4 |
| Untreated | 0 | 0 | 0 | 0 | 0 | 0 | 0 |
| Comparison | | | | | | | |
| Isoproturon | 3 | 3 | 4 | 4 | 4 | 4 | 4 |

**Example F**

In a greenhouse, the noted plant species were treated pre-emergently with the noted compound, at a rate of 0.3 kg active ingredient/ha. The compound was sprayed evenly over the soil as emulsions or suspensions in 500 litres water/ha. Three weeks after the treatment, the compound of the invention showed a high crop selectivity in cotton and maize with excellent activity against the weeds. The comparison material did not show the same high activity.

| Compound No. | GOSHI | ZEAMX | GALAP | VERPE |
|---|---|---|---|---|
| 28 | 0 | 0 | 3 | 4 |
| Untreated | 0 | 0 | 0 | 0 |
| Comparison | | | | |
| Diuron | 0 | 1 | 2 | 2 |

**Claims**

1. A herbicidal composition which comprises a substituted 1,2,4-triazolo-[4,3-a]pyridine of general formula I

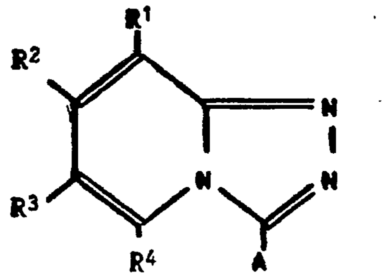

(I)

or a substituted N'-(2-pyridyl)hydrazone of general formula II

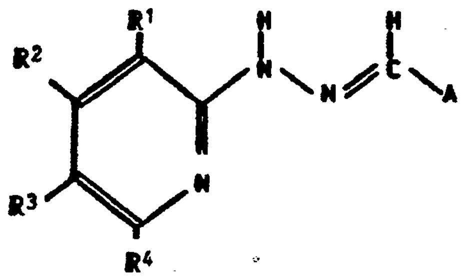

(II)

in which

A is a 5-membered heterocyclic group, comprising 1 to 3 hetero atoms selected from N, O and S, in which any ring carbon is optionally substituted by halogen, $C_{1-4}$-alkyl or halo-$C_{1-4}$-alkyl and one ring nitrogen is optionally substituted by $C_{1-4}$-alkyl, or

A is $C_{1-10}$-alkyl, $C_{2-10}$-alkenyl or $C_{2-10}$-alkynyl, each of which is optionally substituted by one or more of the same or different halogen, cyano, $C_{3-6}$-cycloalkyl, halo-$C_{3-6}$-cycloalkyl, cyano-$C_{3-6}$-cycloalkyl, $C_{1-4}$-alkyl-$C_{3-6}$-cycloalkyl, halo-$C_{1-4}$-alkyl-$C_{3-6}$-cycloalkyl, cyano-$C_{1-4}$-alkyl-$C_{3-6}$-cycloalkyl, $C_{3-6}$-cycloalkenyl, halo-$C_{3-6}$-cycloalkenyl, cyano-$C_{3-6}$-cycloalkenyl, $C_{1-4}$-alkyl-$C_{3-6}$-cycloalkenyl, halo-$C_{1-4}$-alkyl-$C_{3-6}$-cycloalkenyl or cyano-$C_{1-4}$-alkyl-$C_{3-6}$-cycloalkenyl, $C_{3-8}$-cycloalkyl or $C_{3-8}$-cycloalkenyl, each of which is optionally substituted by one or more of the same or different halogen, cyano, $C_{1-4}$-alkyl, halo-$C_{1-4}$-alkyl, cyano-$C_{1-4}$-alkyl, $C_{2-4}$-alkenyl, halo-$C_{2-4}$-alkenyl, cyano-$C_{2-4}$-alkenyl, $C_{2-4}$-alkynyl, halo-$C_{2-4}$-alkynyl or cyano-$C_{2-4}$-alkynyl, phenyl or benzyl, each of which is optionally substituted by one cr more of the same or different halogen, hydroxy, $C_{1-6}$-alkyl, halo-$C_{1-6}$-alkyl, $C_{3-6}$-cycloalkyl, halo-$C_{3-6}$-cycloalkyl, phenyl-$C_{1-6}$-alkyl, $C_{2-6}$-alkenyl, halo-$C_{2-6}$-alkenyl, phenyl-$C_{2-6}$-alkenyl, $C_{2-6}$-alkynyl, halo-$C_{2-6}$-alkynyl, phenyl-$C_{2-6}$-alkynyl, $C_{1-6}$-alkoxy, halo-$C_{1-6}$-alkoxy, $C_{1-4}$-alkoxy-$C_{1-6}$-alkoxy, $C_{1-4}$-alkylamino-$C_{1-6}$-alkoxy, di-$C_{1-4}$-alkylamino-$C_{1-6}$-alkoxy, $C_{2-6}$-alkenyloxy, mercapto, $C_{1-6}$-alkylthio, halo-$C_{1-6}$-alkylthio, $C_{1-6}$-alkylsulphinyl, $C_{1-6}$-alkylsulphonyl, $C_{1-6}$-alkanoyl, halo-$C_{1-6}$-alkanoyl, $C_{1-6}$-alkoxycarbonyl, phenyl, halophenyl, $C_{1-6}$-alkylphenyl, phenoxy, halophenoxy, $C_{1-6}$-alkylphenoxy, nitrophenoxy, amino, $C_{1-4}$-alkylamino, di-$C_{1-4}$-alkylamino, anilino, haloanilino, $C_{1-6}$-alkylanilino, cyano, nitro, methylenedioxy, ethylenedioxy, isopropylidenedioxy, or naphthyl or naphthylmethyl, each of which is optionally substituted by one or more of the same or different halogen, hydroxy, $C_{1-4}$-alkyl, $C_{1-4}$-alkoxy, amino, $C_{1-4}$-alkylamino, di-$C_{1-4}$-alkylamino, cyano or nitro,

$R^1$ is halogen, hydroxy, $C_{1-6}$-alkyl, halo-$C_{1-6}$-alkyl, $C_{1-6}$-alkoxy, halo-$C_{1-6}$-alkoxy, $C_{1-6}$-alkylthio, halo-$C_{1-6}$-alkylthio, $C_{1-6}$-alkylsulphinyl, $C_{1-6}$-alkylsulphonyl, $C_{1-6}$-alkoxycarbonyl, phenyl-$C_{1-6}$-alkoxycarbonyl, phenyl, phenoxy, $C_{1-4}$-alkylamino, di-$C_{1-4}$-alkylamino, nitro or cyano, and

$R^2$, $R^3$ and $R^4$, which may be the same or different, have the same meaning as $R^1$, or are hydrogen,

said compound being in admixture with an agriculturally acceptable diluent or carrier.

**2.** Compounds of formula I as disclosed in claim 1, in which A and $R^{1-4}$ are as defined in claim 1, with the proviso that
a) when A is phenyl, $R^1$ is not bromo or methyl,
b) when A is 4-methoxyphenyl, $R^1$ is not nitro,
c) when A is methyl, $R^1$ is not bromo, amino, nitro or methyl, or
d) when A is ethyl, $R^1$ is not methyl.

**3.** Compounds of formula II as disclosed in claim 2, in which A and $R^{1-4}$ are as defined in claim 2, with the proviso that
a) when A is phenyl, $R^1$ is not bromo,
b) when A is phenyl and $R^2$ is methyl, $R^3$ is not nitro, or
c) when A is phenyl and $R^1$ and $R^3$ are chloro, $R^4$ is not methyl.

**4.** Compounds of formula I, according to claim 2 or 3 in which
$R^1$ is halogen or trifluoromethyl;
$R^2$ is hydrogen;
$R^3$ is hydrogen, trifluoromethyl or halogen;
$R^4$ is hydrogen or halogen;
A is thienyl, furyl, pyrrolyl, imidazolyl or 1,2,3-thiadiazolyl, each of which is optionally substituted by $C_{1-4}$-alkyl, preferably methyl; $C_{4-6}$-alkyl, $C_{3-5}$-alkenyl or $C_{3-8}$-cycloalkyl, each of which is optionally substituted by halogen, especially chlorine; phenyl or benzyl, each of which is optionally substituted by one or more of the same or different halogen, nitro, methyl, methylthio, methoxy, trifluoromethyl, methoxycarbonyl, dimethylamino, phenyl or phenoxy; or is naphthyl.

**5.** Compounds according to claim 4, in which
$R^1$ is bromine or trifluoromethyl;
$R^2$ and $R^4$ are hydrogen;
$R^3$ is hydrogen or bromine; and
A is 2- or 3-thienyl, optionally substituted by methyl, 5-methylfuryl, N-methylpyrrolyl or 4-methyl-1,2,3-thiadiazolyl.

**6.** A method of combating weeds which comprises applying to the weeds or their locus a compound as disclosed in any one of claims 1 to 5.

**7.** A herbicidal composition which comprises a compound claimed in any one of claims 2 to 5, in admixture with an agriculturally acceptable diluent or carrier.